# EUROPEAN PATENT SPECIFICATION

(11) **EP 0 934 343 B1**
(45) Date of publication and mention of the grant of the patent: **08.01.2003**
(21) Application number: 97944051.8
(22) Date of filing: 22.10.1997
(51) Int. Cl.: C08B 37/14, A61K 7/06, C11D 3/22

(54) **DERIVATIZED GUAR GUM COMPOSITION INCLUDING NON-IONIC AND CATIONIC GROUPS WHICH DEMONSTRATE EXCELLENT SOLUTION CLARITY PROPERTIES FOR DETERGENT APPLICATIONS**
ZUSAMMENSETZUNG AUF BASIS VON DERIVATISIERTEM GUARGUMMI, WELCHE NICHTIONISCHE UND KATIONISCHE GRUPPEN ENTHÄLT, UND DIE DARAUS HERGESTELLTEN LÖSUNGEN SEHR KLAR UND IN REINIGUNGSMITTELN VERWENDBAR SIND
COMPOSITION DE GOMME DE GUAR DERIVATISEE COMPRENANT DES GROUPES NON IONIQUES ET CATIONIQUES, PRESENTANT D'EXCELLENTES PROPRIETES DE LIMPIDITE EN SOLUTION, DESTINEE A DES APPLICATIONS DE DETERGENTS

(30) Priority: 25.10.1996 US 736866; 25.10.1996 US 738290
(43) Date of publication of application: 11.08.1999
(73) Proprietor: RHODIA INC., Cranbury, New Jersey 08512-7500 (US)
(72) Inventor: CHOWDHARY, Manjit, Singh, Princeton Juction, NJ 08550 (US); ROBINSON, Fred, Newtown, PA 18940 (US)
(74) Representative: Fabre, Madeleine-France
(86) International application number: IB9701325
(87) International publication number: WO98018828

(56) References cited:
- EP-A- 0 331 472
- EP-A- 0 686 643
- PATEL S P ET AL: "SYNTHESIS AND CHARACTERIZATION OF QUATERNARY AMMONIUM COMPOUNDS OF GUAR GUM AND HYDROXYETHYL GUAR GUM" STARCH STARKE, vol. 41, no. 5, May 1989, pages 192-196, XP000026584
- PATENT ABSTRACTS OF JAPAN vol. 95, no. 2, 31 March 1995 & JP 06 312915 A (TSUMURA & CO), 8 November 1994,
- PATENT ABSTRACTS OF JAPAN vol. 8, no. 205 (C-243), 19 September 1984 & JP 59 096103 A (MITSUBISHI ACETATE), 2 June 1984,

## Description

The present invention relates to polygalactomannan compositions and more particularly derivatized guar gum compositions including cationic groups which, when dispersed in water or other solvents to yield a highly viscous solution, are capable of forming a relatively transparent solution. The compositions are particularly useful for applications where clarity and purity of aqueous solutions are desirable such as oil recovery, personal care products, textile chemicals, paper chemicals, paints, and the like.

### 2. Technology Description

Natural and synthetic polymers containing hydroxy groups have been used as thickeners for foods, coatings, paints, explosive slurries, oil well fluids, cosmetics and other personal care products, and many other functional applications.

One class of polymers that have been widely used as suspending and viscosity agents are polygalactomannans. Polygalactomannans are polysaccharides composed principally of galactose and mannose units and are usually found in the endosperm of leguminous seeds such as guar, locust bean, honey locust, flame tree, and the like. The polygalactomannans may be used in either their natural form or may be substituted with one or more functional groups (e.g., carboxymethyl group). The most commonly used polygalactomannan is guar gum. In practice, to thicken a fluid the polygalactomannans may either be added by themselves, or with other viscosity modifiers such as other polysaccharides, xanthan gum and the like.

While the use of polygalactomannans, and guar gum in particular, as thickening agents has been met with great success, it is still desired to improve the physical properties of the guar gum when dispersed in a solution such as water. One such property is its ability to transmit light when used as an aqueous solution, and particularly one having a high viscosity having uses in personal care applications such as shampoos. Aqueous solutions of guar gum tend to be opaque or translucent at best. It is particularly desired that clear, colorless and less impure solutions be produced when dispersing guar gum in water as it makes the final solution more useful for the above-described applications.

U.S. Patent No. 4,693,982 discloses the use of enzymes to reduce insolubles in guar gum. The level of clarity produced by the enzymatic treatment is not disclosed in the patent.

U.S. Patent No. 4,874,854 discloses low viscosity heteropolysaccharides, for example, guar gum. Example 3 discloses a "clarified" guar gum which is produced by cold filtration of a 0.3% solution through diatomaceous earth and precipitated with isopropyl alcohol. The ability of this material to transmit light is not disclosed in the patent.

U.S. Patent No. 3,912,713 discloses non-lumping derivatives of guar gum produced by derivatizing guar gum splits at a moisture content of 20-80% by weight, raising the moisture content of the splits, if necessary, to 30-80% by weight and fragmenting the splits by pressing them out in a thin layer and drying them on a cylinder heated to 100-180°C and comminuting the film to form particulates of a size in the order of +20 mesh, as measured by a Tyler screen, and preferably having a size of 2-5 mm. This process is commonly referred to as drum drying. According to Example 3, the product produced in the Example gives a "clear solution having no lumps or clots on stirring in water". No quantitative definition of "clear" (i.e. light transmittance) is presented in the patent.

U.S. Patent No. 4,057,509 discloses polygalactomannan allyl ether gels. According to Example 1, guar gum is purified to yield a material having less than 0.1% nitrogen content and about 0.48% ash. The same purification method is described in U.S. Patent No. 4,169,945.

U.S. Patent No. 4,659,911 teaches the treatment of 100 parts of guar gum with at least 150 parts of an aqueous alkali solution whereby the water present in the entire solution exceeds 60%. No data regarding the ability of resulting aqueous solutions to transmit light is set forth.

Commonly owned U.S. Patent Nos. 5,489,674 and 5,536,825 relate to a method for treating polygalactomannan splits to yield a product which, when dissolved in solution at 0.5 weight percent, is able to only provide light transparency, i.e., of greater than 75% at 500-600 nm. The method includes treating the splits in an aqueous alkaline solution followed by washing the splits in water or an organic solvent.

U.S. Patent Nos. 3,326,890; 3,350,386; and 3,483,121 are directed to methods for producing hydroxyalkyl polygalactomannans by using guar flour as a starting material. Included amongst the teachings is the suspension of guar flour in an organic, water-miscible solvent, and adding an alkaline material and an alkylene oxide to yield the final material.

For use in personal care applications such as soaps, body washes, shampoos and the like, it has been demonstrated that excellent performance benefits can occur when using polymeric materials containing cationic groups. This is particularly true in the case of clear personal compositions having increased viscosities (i.e., greater than about 500 centipoises).

Typically such personal care applications include inorganic salts such as NaCl or NH₄Cl to provide viscosity to the final composition. The use of NaCl is preferred because it provides added viscosity at a low cost without any off odor while being environmentally friendly. For use in combination with NaCl containing personal care products, a preferred cationic polymer has been quaternized hydroxyethyl cellulose. The use of cationic polygalactomannans would be particularly preferred because of their combination of thickening and environmentally friendly properties. However, it has been impossible to use cationic polygalactomannans in the past in conjunction with personal care products including inorganic salts because, when added, they produce a cloudy appearance, making them commercially undesirable.

Accordingly, there exists a need in the art for a derivatized guar gum, particularly one having cationic groups which is capable of producing nearly pure, clear and colorless solutions upon dispersing in water and a novel process for producing the gum.

### Brief Summary of the Invention

In accordance with the present invention, cationically derivatized polygalactomannans which are capable of producing nearly pure, clear and colorless solutions upon dispersing in water or an organic solvent, especially those including NaCl, and a novel process for producing them is provided.

One embodiment of the present invention comprises nonionically and cationically derivatized polygalactomannans which demonstrate greater than 75% light transmission at a wavelength of from 500-600 nanometers when dispersed in water in the amount of 0.5 parts per 100 parts water wherein the cationically derivatized polygalactomannans are produced by the process comprising the steps of:
(a) contacting nonionically derivatized guar flour with an alcohol or an alcohol/water solution;
(b) neutralizing the reaction mixture of step (a) with an acid;
(c) adding a cationic substituent in an amount such that the resulting material has a degree of substitution of the cationic substituent ranging from 0.20 to 1.0;
(d) adding an aqueous alkaline solution;
(e) washing the resulting mixture with water, an organic solvent or mixtures thereof; and
(f) recovering the product produced thereby.

In particular embodiments, the polygalactomannan produced includes both nonionic groups and cationic groups having specific molecular substitution and degrees of substitution respectively. In still other embodiments, the polygalactomannan produced may also include anionic groups, yielding an amphoteric material.

Another embodiment of the present invention comprises an oil field chemical, personal care chemical, industrial cleaning chemical, textile chemical, paper chemical or coating composition including the above-described cationically derivatized guar gum.

A third embodiment of the present invention comprises a process for producing a polygalactomannan comprising the steps of:
(a) contacting nonionically derivatized guar flour with an alcohol or an alcohol/water solution;
(b) neutralizing the reaction mixture of step (a) with an acid;
(c) adding a cationic substituent in an amount such that the resulting material has a degree of substitution of the cationic substituent ranging from 0.20 to 1.0;
(d) adding an aqueous alkaline solution;
(e) washing the resulting mixture with water, an organic solvent or mixtures thereof; and
(f) recovering the product produced thereby.

Still another embodiment of the present invention comprises a detergent composition useful for cleaning and/or conditioning human hair or skin or cleaning dishes or clothing which contains NaCl or NH₄Cl and further contains a nonionically and cationically derivatized polygalactomannan, the molecular substitution of the nonionic substituent being between 0.4 and 1.2 and the degree of substitution of the cationic substituent being between 0.20 to 1.0, said composition having a viscosity of greater than 500 centipoises and said composition having greater than 75% light transmission at a wavelength of from 500-600 nanometers.

Still another embodiment of the present invention comprises a detergent composition useful for cleaning and/or conditioning human hair or skin or cleaning dishes or clothing which contains NaCl or NH₄Cl and further contains a nonionically, anionically and cationically derivatized polygalactomannan, the molecular substitution of the nonionic substituent being between 0.14 and 1.2, the degree of substitution of the anionic substituent being between 0.10 to 0.30 and the degree of substitution of the cationic substituent being between 0.20 to 1.0, said composition having a viscosity of greater than 500 centipoises and said composition having greater than 75% light transmission at a wavelength of from 500-600 nanometers.

Accordingly, it is an object of the present invention to provide a nonionically and cationically derivatized polygalactomannan which easily transmits light when used in an aqueous solution, has a low amount of insolubles, demonstrates excellent crosslinking properties, and can maintain a constant solution viscosity for an extended period of time.

It is another object of the present invention to provide an oil field chemical, food product, personal care or cosmetic chemical, absorbent material, textile chemical, paper chemical or coating composition which includes the novel derivatized polygalactomannan.

A further object of the present invention is to provide a process for making the derivatized polygalactomannan.

A still additional object of the present invention is to provide a detergent composition having excellent solution clarity and high viscosity.

These, and other objects, will readily be apparent to those skilled in the art as reference is made to the detailed description of the preferred embodiment.

### Detailed Description of the Preferred Embodiment

In describing the preferred embodiment, certain terminology will be utilized for the sake of clarity. Such terminology is intended to encompass the recited embodiment, as well as all technical equivalents which operate in a similar manner for a similar purpose to achieve a similar result.

The present invention relates to novel polygalactomannans and the process for their manufacture. Polygalactomannans are polysaccharides composed principally of galactose and mannose units and are usually found in the endosperm of leguminous seeds, such as guar, locust bean, honey locust, flame tree, and the like. Guar flour, for example, is composed mostly of a galactomannan which is essentially a straight chain mannan with single membered galactose branches. The mannose units are linked in a 1-4-β-glycosidic linkage and the galactose branching takes place by means of a 1-6 linkage on alternate mannose units. The ratio of galactose to mannose in the guar polymer is, therefore, one to two.

Locust bean gum is also a polygalactomannan gum of similar molecular structure in which the ratio of galactose to mannose is one to four. Guar and locust bean gum are the preferred sources of the polygalactomannans, principally because of the commercial availability thereof. In the preferred embodiment, the invention is directed to the production of guar gum.

The preferred guar starting material used in this invention is guar flour, which is often also referred to as guar powder, which has been derivatized with a nonionic substituent.

Preferred nonionic substituents are hydroxyalkyl, wherein alkyl represents a straight or branched hydrocarbon moiety having between 1 and 6 carbon atoms (e.g., hydroxyethyl, hydroxypropyl, hydroxybutyl, etc.). The substituents are linked to the guar molecule by the reaction of the guar molecule with alkylene oxides (e.g., ethylene oxide, propylene oxide or butylene oxide). This reaction can take place when the guar is in the ''splits" or "flour" form and is considered well known by those skilled in the art.

The term "molar substitution" as employed herein is the average number of moles of functional groups per anhydro sugar unit in the polygalactomannan gum. A particularly desired molar substitution of hydroxyalkyl groups is between 0.01 and 3.0, more preferably between 0.14 to 1.20. If the final polymer produced is cationic in nature, the molar substitution of the hydroxyalkyl groups should be between 0.40 to 1.20, even more preferably between 0.40 to 0.80 whereas if the final polymer produced is amphoteric in nature, the molar substitution of the hydroxyalkyl groups should be between 0.14 to 1.20, even more preferably between 0.14 to 0.80. Preferred functional groups are the hydroxyethyl and hydroxypropyl groups.

Also considered within the scope of the invention is to use a starting material which includes one or more anionic groups. In practice the anionic substituent will be a carboxyalkyl group wherein alkyl represents a straight or branched hydrocarbon moiety having between 1 and 6 carbon atoms. Examples of such substituents include carboxymethyl, carboxyethyl and carboxypropyl groups and the like having a degree of substitution of between 0.01 and 3.0. Particularly preferred is the use of a carboxymethyl substituent having a degree of substitution of between 0.1 and 0.3.

The term "degree of substitution" as employed herein is the average substitution of functional groups per anhydro sugar unit in the polygalactomannan gum. In guar gum, the basic unit of the polymer consists of two mannose units with a glycosidic linkage and a galactose unit attached to the C6 hydroxyl group of one of the mannose units. On the average, each of the anhydro sugar units contains three available hydroxyl sites. A degree of substitution of three would mean that all of the available hydroxyl sites have been esterified with functional groups.

The above starting materials, and more specifically hydroxypropyl guar gum powder and carboxymethylhydroxypropyl guar gum are commonly available from manufacturers such as Rhône-Poulenc Inc. These materials are considered the starting materials for the present invention.

The starting nonionically substituted guar gum, or nonionically and anionically substituted guar gum in powder form, is then treated with an alcohol or an alcohol/water solution. In practice, the alcohol selected may be methanol, ethanol, isopropanol, n-propyl alcohol, n-butyl alcohol and the like.

The alcohols may be used in neat (100%) form or in combination with water, the primary consideration being that the alcohol is miscible with water. Treatment is typically made at ambient temperatures, for example, about30°C, although the temperature can be increased up to about70°C while still obtaining efficacious results.

In practice it is generally preferred to use mixtures of water and organic solvents in this step. Particularly effective results occur when using between 10 to 90 percent water by weight and between 90 to 10 percent organic solvent by weight. Most preferred is the use of between 80 to 90% by weight isopropyl alcohol and between 10 to 20% by weight water. For example, excellent results have been obtained wherein the solution selected for use is an isopropanol/water mixture, wherein the respective amounts by weight are 85% isopropanol and 15% water.

The amount of alcohol or alcohol water solution to be used in this step is that amount which is necessary to fully saturate the guar powder. In practice, this amount is usually at least twice the amount by weight of the starting guar powder, and even more preferably, at least three times the amount by weight of the starting guar powder.

The resulting material will typically be alkaline. To improve the clarity obtained when using the final product of the present invention, the alkaline material should be neutralized to a neutral pH (e.g., pH about 7). Any acid may selected for use to neutralize the solution, including strong acids such as hydrochloric acid and sulfuric acid or weak acids such as acetic acid. In a preferred embodiment either hydrochloric or acetic acid is used. The amount of acid used is the amount necessarily for neutralization. Determination of this amount is accomplished by a simple acid/base analysis and is considered well within the skill in the art.

Treatment is typically made at ambient temperatures, for example, about 30°C.

After addition of the alcohol or alcohol/water solution and the subsequent neutralization, followed by agitation as needed, a cationic reagent is added so that the guar gum is derivatized with one or more cationic groups. Cationic substituents include primary, secondary, or tertiary amino groups or quaternary ammonium, sulfonium or phosphinium groups.

Illustrative cationic groups suitable for the practice of the present invention include quaternary ammonium groups. Typical of quaternary ammonium groups are tetramethylammonium chloride and bromide, benzyltrimethylammonium chloride and bromide, tetraethylammonium chloride and bromide, tetrabutylammonium chloride and bromide, methylpyridinium chloride and bromide, benzylpyridinium chloride and bromide, trimethyl-p-chlorobenzylammonium chloride and bromide, and the like, wherein each of the said groups is derivatized in the form of a radical which is substituted in a hydrocolloid gelling agent by means of an alkylene or oxyalkylene linkage.

In preferred embodiments, the quaternary ammonium ether substituents correspond to the formula 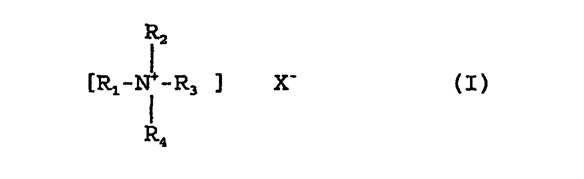 wherein R₁ is a monohydroxylated or polyhydroxylated alkyl group containing between one and six carbon atoms; R₂ and R₃ are independently, alkyl groups containing between one and six carbon atoms; R₄ is an alkyl group containing between one and 24 carbon atoms; and X is a halide. The said alkyl groups can contain other atoms such as oxygen, sulfur and halogen.

Specific substituents include 2,3-epoxypropyl N,N,N-trimethylammonium chloride, 3-chloro-2-hydroxypropyl N,N,N-dodecyldimethylammonium chloride, 3-chloro-2-hydroxypropyl N,N,N-cocoalkyldimethylammonium chloride, and 3-chloro-2-hydroxypropyl N,N,N-octadecyldimethylammonium chloride. Such chemicals are commercially available from The Degussa Chemical Company under the trade names QUAB-151, QUAB-342, QUAB-360 and QUAB-426 respectively.

The amount of cationic reagent added to the guar powder is the amount that yields a cationic degree of substitution ranging from 0.20 to 1.0, and even more preferably between 0.23 and 0.80. Calculation of these amounts is easily obtained by simple stoichiometric equations and is considered well within the skill of the artisan.

To improve the reaction kinetics of the cationic substitution, an alkaline material is added as a catalyst. The alkaline material is typically aqueous solution of base, preferably an aqueous solution containing greater than 12% of NaOH, and more preferably a 12 to 60% aqueous solution of NaOH. Even more preferred is treatment with a 20% to a 50% aqueous solution. In the preferred embodiment between 2 and 50 parts of NaOH (100%) are added for every 100 parts of starting guar flour.

Thereafter, the temperature of the reaction mixture is increased to between 40 and 75°C, with a reaction temperature being between 60 and 70°C being even more preferred, and the mixture is stirred for a period of time sufficient to insure complete reaction of the reactants. In practice, this reaction time is between about one and about five hours, with a time of about three hours being more preferred.

Thereafter, the resulting material is neutralized to a neutral pH (e.g., pH about 7). Any acid may selected for use to neutralize the solution, including strong acids such as hydrochloric acid and sulfuric acid or weak acids such as acetic acid. In a preferred embodiment either hydrochloric or acetic acid is used. The amount of acid used is the amount necessarily for neutralization. Determination of this amount is accomplished by a simple acid/base analysis and is considered well within the skill in the art.

After the neutralization, the derivatized guar powder is filtered and then washed with water, an organic solvent, or a mixture of both. Particularly preferred organic solvents are water miscible solvents including alcohols such as methanol, ethanol, isopropanol, n-propyl alcohol, n-butyl alcohol and the like. Other commonly used purification solvents such as acetone, glycols and the like may alternatively be selected. Of the above solvents, the most preferred is the use of methanol, ethanol, isopropyl alcohol, acetone and mixtures thereof.

The volume of the wash liquid is much greater than the amount of treated flour and can be performed in batchwise or multiple applications. In preferred practices the volume of the wash liquid is at least twice the volume of the treated flour and preferably at least three times the volume of the flour. It is preferable to conduct between 2 to 4 independent wash cycles. The concentration of the wash liquid for each cycle can be the same or different. For example washing of the powder may be accomplished by first washing with a dilute isopropyl alcohol solution, followed by washing with stronger solutions of isopropyl alcohol, and finally with acetone.

After washing, the derivatized guar powders are then dried and recovered using means known in the art. Examples of such means include air drying, filtering, evaporative drying, centrifuging, flash grinding, addition of solvents, freeze drying and the like. The use of fluidized bed drying is particularly recommended.

The resulting materials are particularly advantageous in that they are capable of achieving very high clarity when added to water in solution form. When added to water in 0.5% solutions, the inventive materials are capable of producing light transmissions of greater than 75% at 500-600 nm, with water being 100%. By using the inventive process, the guar gum produced can have a light transmission (0.5% solution) of greater than 80%, more preferably greater than 90%, and most preferably between 93% and 100% (with water being 100%).

The compositions are also capable of producing high viscosity solutions while maintaining their clarity. For example a one percent solution of the inventive composition can yield a viscosity of between 300 and 1000 centipoises using the above process. Increased viscosities can be obtained by conducting the reactions in an inert (e.g., nitrogen) environment.

Because of these above properties, the inventive materials are suitable for a wide range of commercial applications. Included amongst them are in oil and gas recovery, because of the low amount of enzymatic hydrolysis residues, high crosslinking ability, ease of filtration during natural gas recovery and potential synergy with other components such as cross-linking agents; personal care and industrial detergent products, because of low protein content, potentially improved skin sensitivity, clear and colorless appearance for consumer appeal, potential compatibility with other surfactants and personal care chemicals such as polyorganosiloxanes and potential flow modification characteristics; textile chemicals because of potential improved jet flow in textile machinery and increased molecular interaction with xanthan gum; dyes; paper chemicals including print and processing chemicals; paints; food products; explosives; absorbent materials; agricultural products; cosmetics; and any other application where the above combination of high clarity with high viscosity would be beneficial.

In addition, when crosslinked with agents such as borax as discussed above, the inventive compositions are able to provide extremely high crosslinked gels.

A particularly preferred use of the inventive compositions is in personal care compositions, more preferably in shampoos, body washes and in industrial cleaning compositions such as dishwashing detergents, laundry detergents and the like, where the thickening and light transmittance properties of the compositions can advantageously be utilized. In such formulations, the amount of the nonionic and cationic derivatized polygalactomannan to be included is preferably between 0.1 and 2.0 percent by weight of the formulation, with amounts between 0.3 and 1.5 percent being more preferred and amounts between 0.5 and 1.0 percent by weight being most preferred.

In detergent compositions, the formulations used can typically include one or more surfactants in an aqueous carrier. The surfactants selected for use in producing such formulations are considered within the skill of the artisan and can be selected from nonionic, anionic, cationic, amphoteric and zwitterionic surfactants known in the art. Mixtures of the above surfactants may also be selected.

Examples of nonionic surfactants which may be selected include fatty acid amides, alkoxylated fatty alcohol amines, fatty acid esters, glycerol esters, alkoxylated fatty acid esters, sorbitan esters, alkoxylated sorbitan esters, alkylphenol alkoxylates, aromatic alkoxylates and alcohol alkoxylates.

Examples of anionic surfactants which may be selected include alkyl sulfates (alkyl is a fatty alkyl or alkylaryl group), ether sulfates, alkyl sulfonates, sulfosuccinates, sulfosuccinamates, naphthalene formaldehyde condensates, isethionates, taurates, phosphate esters and ether carboxylates.

Examples of cationic surfactants which may be selected include cationic quaternaries such as imidazolines arylalkyl quaternary compounds and aromatic quaternary compounds, amine oxides, and alkoxylated amines.

Examples of amphoteric surfactants which may be selected include betaines, sultaines, glycinates, amphoteric imidazoline derivatives and aminopropionates.

All of the above types of surfactants are commercially available and sold by Rhône-Poulenc Inc.

Also commonly included is an inorganic salt such as NaCl or NH₄Cl to provide thickness to the final formulation. The use of NaCl is preferred because it has an extremely low cost. NaCl is typically present in amounts greater than 0.5 percent by weight of the final formulation, and typically between 0.5 to 5.0 percent by weight of the final formulation, more preferably between 1.0 to 3.0 percent by weight of the final formulation. Also expressly considered as falling within the scope of the present inventions are products which utilize NH₄Cl or other inorganic salts in place of NaCl.

However, a significant problem that has occurred in the past when trying to add cationic polygalactomannans to the final formulation of a personal care composition is that the presence of the NaCl (or NH₄Cl) interacts with the cationic polygalactomannan resulting in an opaque solution. For aesthetic and market reasons, it is highly desirable to produce a clear formulation. It has been surprisingly discovered that the addition of the cationic polygalactomannans according to the present invention retain a clear appearance while providing added thickness properties. In addition, these materials are biodegradable, environmentally friendly, and mild to human hair and skin. Further, they can enable the production of a superior performing product. For example, shampoos in accordance with the present invention demonstrate superior combing and compatibility results as compared with those using commercially successful cationic polymers.

It has further been surprisingly discovered that only those nonionic and cationic derivatized polygalactomannans having the molecular substitution of the nonionic substituent being between 0.4 and 1.2 and the degree of substitution of the cationic substituent being between 0.20 to 1.0, or those nonionic, anionic and cationically derivatized polygalactomannans, the molecular substitution of the nonionic substituent being between 0.14 and 1.2, the degree of substitution of the anionic substituent being between 0.10 to 0.30 and the degree of substitution of the cationic substituent being between 0.20 to 1.0 can effectively produce clear solutions when combined with personal care formulations containing NaCl. Compositions which do not have these respective molecular and degree of substitutions yield opaque solutions, making them commercially undesirable.

The formulation may also include optional additives such as perfumes, dyes, natural or synthetic fragrances, conditioning aids, and the like.

The final formulation will have a viscosity of greater than 500 centipoises, preferably between 1000 and about 25000 centipoises and most preferably between 5000 and 20000 centipoises. The final formulation will also preferably have a light transmission of greater than 80%, more preferably greater than 90%, and most preferably between 93% and 100%.

The invention will be better understood by reference to the following examples.

### Example 1

200 grams of JAGUAR® 8060 flour, a hydroxypropyl guar manufactured by Rhône-Poulenc Inc. having a molecular substitution of about 0.4 are added to 1000 ml. of an 85 percent isopropyl alcohol solution. The reaction is stirred for a few minutes and is neutralized with concentrated HCl or glacial acetic acid to neutralize a pH value of 7. Thereafter, 100 gms of 2,3-epoxypropyl N,N,N-trimethylammonium chloride are added over a 15 minute period and the mixture is stirred for an additional 15 minutes. 50 ml of a 50% NaOH solution are added over a 15 minute period and the mixture is stirred for an additional 15 minutes. Thereafter, the mixture is heated to 65°C and is held at 65°C for three hours. The mixture is cooled to about room temperature and is neutralized to a pH of about 7 by the addition of glacial acetic acid or HCl. The mixture is filtered and the filtered solids are washed successively with one liter of 50% isopropyl alcohol aqueous solution, one liter of 85% isopropyl alcohol aqueous solution, one liter of 100% isopropyl alcohol, and one liter of acetone. The solids are then dried in a fluidized bed drier at about 60°C for about one hour.

The resulting composition is a nonionically and cationically derivatized guar gum having a molecular substitution of the nonionic substituent of about 0.4 and a degree of substitution of the cationic substituent of about 0.2. The solution viscosity of a 1% solution at 25°C is 300-1500 cps as measured by a Brookfield Viscometer, 20 rpm and the light transmission of a 0.5% aqueous solution at 500-600 nm is greater than 90%.

### Examples 2-29

The procedure of Example 1 is repeated except that different MS and DS values are obtained. Different molecular substitution (MS) values for the hydroxypropyl substituent are obtained by using different starting hydroxypropyl guar materials (in the case where MS=0, guar gum flour is used). Different degree of substitution (DS) values for the cationic substituent are obtained by using appropriate stoichiometric amounts of QUAB-151. The resulting materials are listed in the following Table.

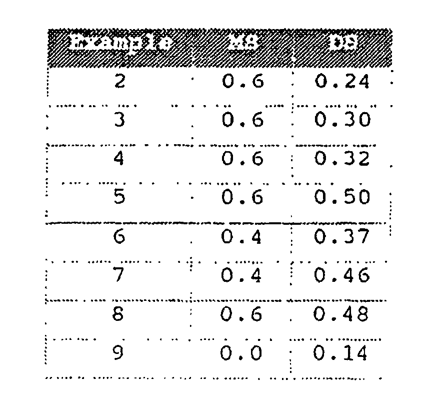

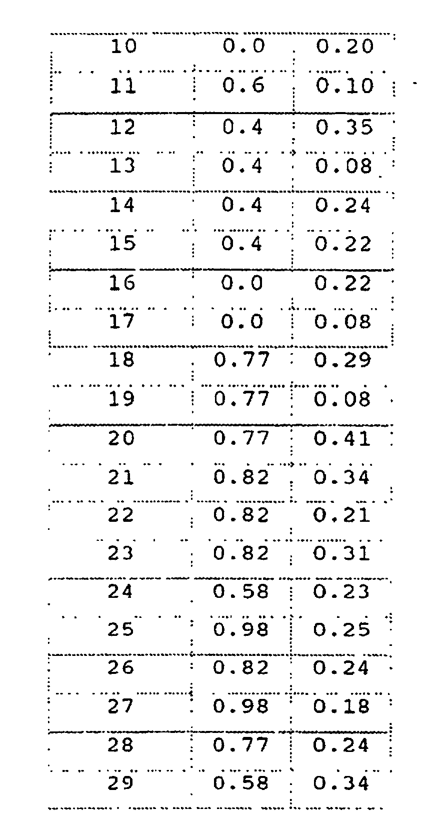

### FORMULATION DATA AND TESTING

Each of the above Example Compositions are used in shampoo formulations. A first formulation includes the following components (amounts are listed by weight):

### Formluation One

| **Component** | **Percent by Weight** |
|---|---|
| Example Composition | 0.5 |
| Sodium Laureth Sulfate (30% active solution) | 10.0 |
| coconut fatty acid amide (100% active) | 2.5 |
| NaCl | 0, 2.0, 2.5 or 3.0 |
| Water | Q.S. |
| TOTAL | 100 |

The viscosity (in centipoises) and light transmittance (600 nm) for each of the formluations are shown in the following table. As a comparative example, a highly commercially successful polymer commonly used in clear shampoos, which is a quaternized hydroxyethyl cellulose having a molecular substitution of hydroxyethyl groups of 1.2 and a degree of substitution of cationic groups of 0.40 is selected for testing. This comparative example is referenced as CE. Each of the tested shampoos effectively functioned in their cleaning ability. However, their desirability would be limited by a low transmittance value, particularly in the presence of NaCl.

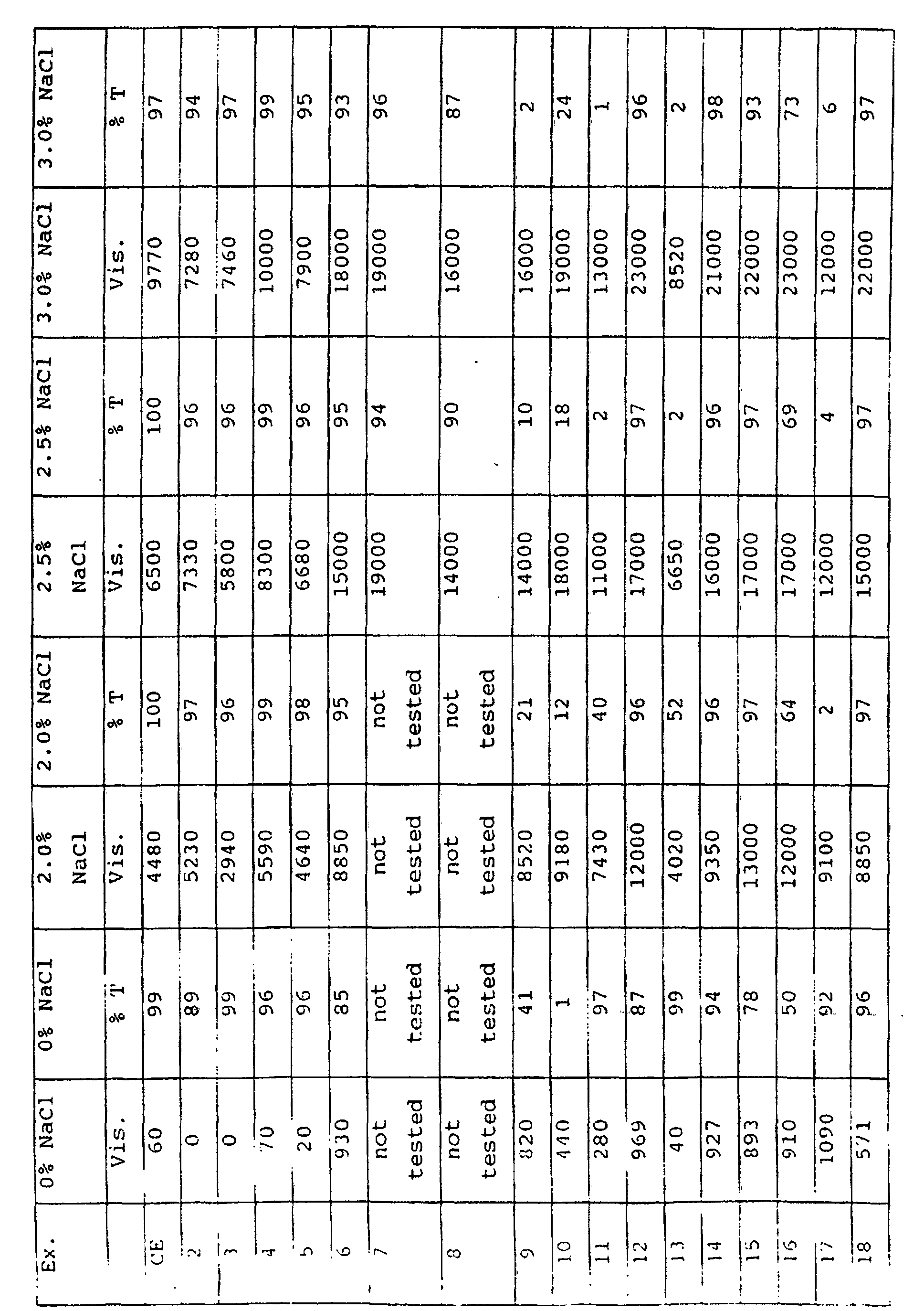

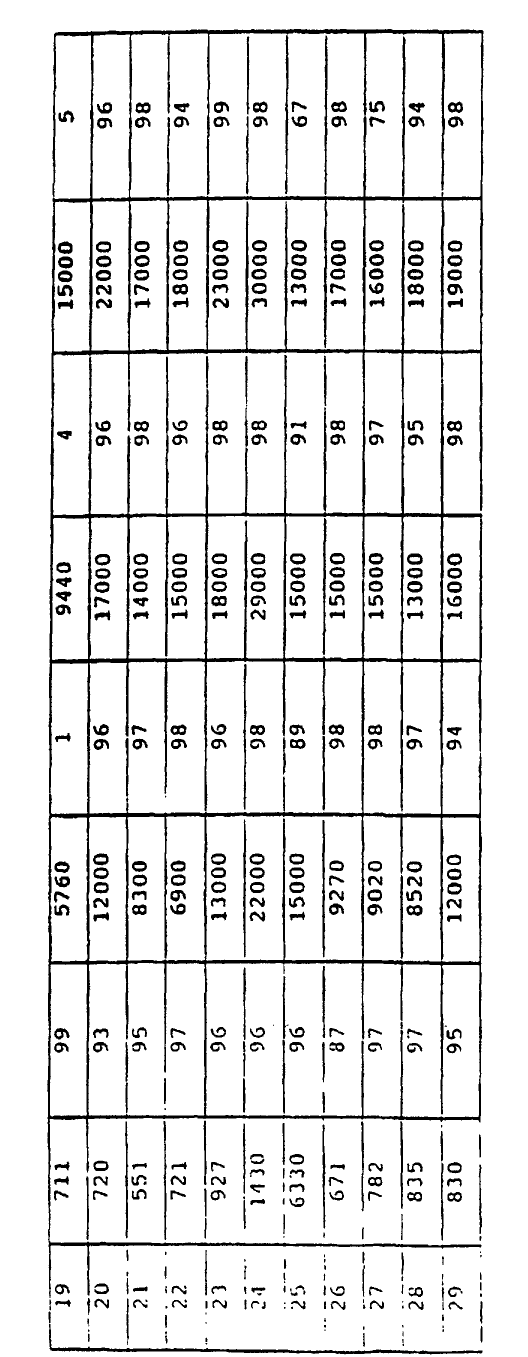

A second formulation includes the following components (amounts are listed by weight):

### Formluation Two:

| **Component** | **Percent by Weight** |
|---|---|
| Example Composition | 0.5 |
| Sodium Laureth Sulfate (30% active solution) | 10.0 |
| cocoamidopropyl betaine (30% active) | 2.5 |
| NaCl | 0, 1.0, 1.5 or 2.0 |
| Water | Q.S. |
| TOTAL | 100 |

The viscosity (in centipoises) and light transmittance (600 nm) for each of the formulations are shown in the following table. As a comparative example, a highly commercially successful polymer commonly used in clear shampoos, which is a quaternized hydroxyethyl cellulose having a molecular substitution of hydroxyethyl groups of 1.2 and a degree of substitution of cationic groups of 0.40 is selected for testing. This comparative example is referenced as CE. Each of the tested shampoos effectively functioned in their cleaning ability. However, their desirability would be limited by a low transmittance value, particularly in the presence of NaCl.

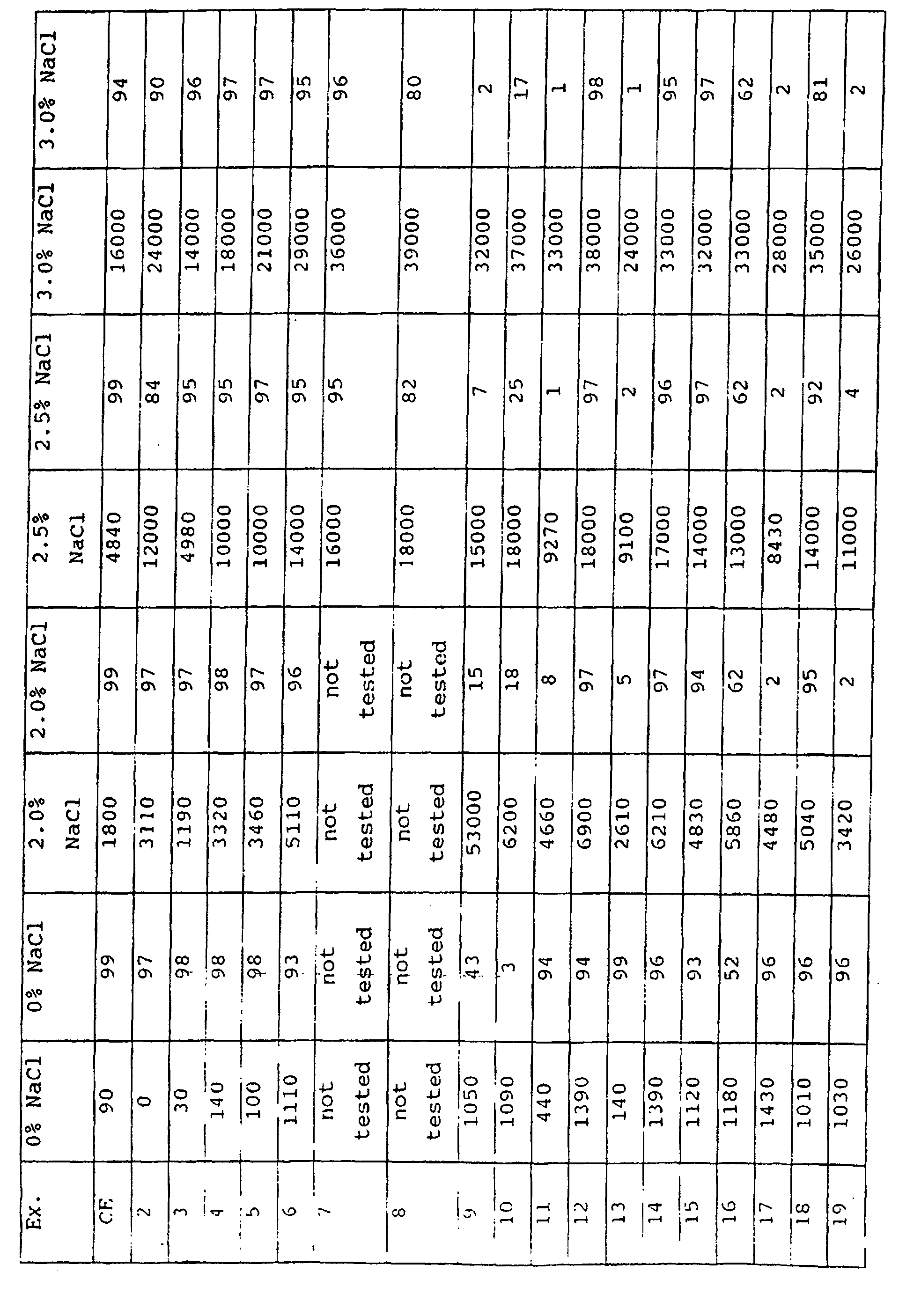

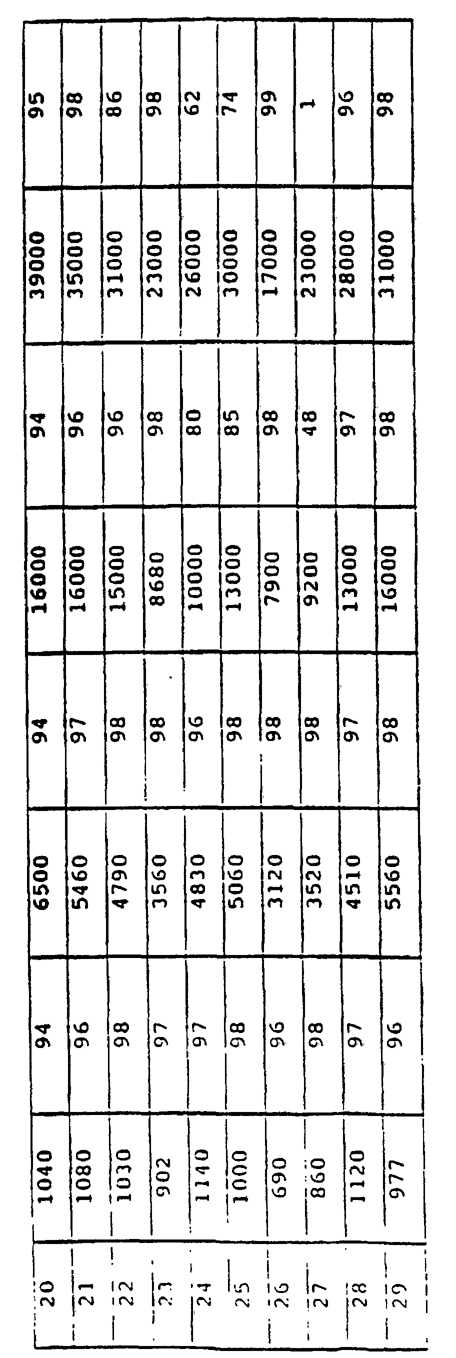

### COMB TESTING

To determine if the formulations would effectively provide functional properties to human hair a Diastrom Wet Combing Test is performed on each of the following Formulation Three Samples.

### Formulation Three:

| Component | Parts by Weight |
|---|---|
| Example Composition | 10.0 |
| Sodium Laureth Sulfate (30% active solution) | 600.0 |
| cocoamidopropyl betaine (30% active) | 199.0 |
| coconut fatty acid amide (100% active) | 169.0 |
| NaCl | 30.0 |
| Formalin | 2.0 |
| Water | Q.S. |
| TOTAL | 2000 |

This industrial standard test measures the difference between the peak force used to comb treated human hair treated with the experimental shampoos as compared to the peak force used to comb human hair which has only been wetted with water. A working area is obtained by measuring the area under the force curve obtained for the test compositions as compared to that of the water only treated hair. A positive value for both peak value and work value is considered desirable. The results are shown in the following Table.

| Sample | Peak Value | Work Area |
|---|---|---|
| CE | 7 | 11 |
| 2 | not tested | not tested |
| 3 | 3 | 7 |
| 4 | 12 | 10 |
| 5 | -1 | 0 |
| 6 | -2 | 0 |
| 7 | 7 | 7 |
| 8 | 5 | -2 |
| 9 | 32 | 32 |
| 10 | 24 | 26 |
| 11 | 31 | 29 |
| 12 | 16 | 17 |
| 13 | 48 | 45 |
| 14 | 12 | 14 |
| 15 | 14 | 12 |
| 16 | 4 | 6 |
| 17 | 49 | 48 |
| 18 | 30 | 26 |
| 19 | 30 | 28 |
| 20 | 3 | -2 |
| 21 | 4 | -2 |
| 22 | 9 | 10 |
| 23 | -7 | -7 |
| 24 | 0 | -1 |
| 25 | 2 | 3 |
| 26 | -8 | -9 |
| 27 | 10 | 12 |
| 28 | 8 | 7 |
| 29 | 6 | 8 |

The above data demonstrates that the molecular substitution of the nonionic substituent being between 0.4 and 1.2 and the degree of substitution of the cationic substituent being between 0.20 to 1.0, yields a composition having a viscosity of greater than 500 centipoises and having greater than 75% light transmission at a wavelength of from 500-600 nanometers. In addition, the resulting personal care compositions have excellent detergency, mildness and combing properties.

### Example 30

200 grams of carboxymethylhydroxypropyl guar manufactured by Rhône-Poulenc Inc. having a molecular substitution of about 0.2 and a degree of substitution of carboxymethyl groups of about 0.1 are added to 1000 ml. of an 85 percent isopropyl alcohol aqueous solution. The reaction is stirred for a few minutes and concentrated HCl or glacial acetic acid is added to neutralize the pH to a value of 7. Thereafter, 120 grams of 2,3-epoxypropyl N,N,N-trimethylammonium chloride are added over a 15 minute period and the mixture is stirred for an additional 15 minutes. 60 ml of a 5% NaOH solution are added over a 15 minute period and the mixture is stirred for an additional 15 minutes. Thereafter, the mixture is heated to 65°C and is held at 65°C for three hours. The mixture is cooled to about room temperature and is neutralized to a pH of about 7 by the addition of concentrated hydrochloric acid or glacial acetic acid. The mixture is filtered and the filtered solids are washed successively with one liter of 50% isopropyl alcohol aqueous solution, one liter of 85% isopropyl alcohol aqueous solution, one liter of 100% isopropyl alcohol, and one liter of acetone. The solids are then dried in a fluidized bed drier at about 60°C for about sixty minutes.

The resulting composition is a nonionically, anionically and cationically derivatized guar gum having a molecular substitution of the nonionic substituent of about 0.2, a degree of substitution of the anionic substituent of about 0.1., and a degree of substitution of the cationic substituent of about 0.4. The solution viscosity of a 1% solution at 25°C is 300-1500 cps as measured by a Brookfield Viscometer, 20 rpm, and the light transmission of a 0.5% aqueous solution at 500-600 nm is greater than 90%. It is believed that this composition could effectively be used in personal care or industrial detergent applications which include NaCl and yield a highly viscous clear product.

Having described the invention in detail and by reference to the preferred embodiments thereof, it will be apparent that modifications and variations are possible without departing from the scope of the appended claims.

## Claims

1. Polygalactomannans which contain both nonionic and cationic groups and demonstrate greater than 75% light transmission at a wavelength of from 500-600 nanometers when dispersed in water in the amount of 0.5 parts per 100 parts water wherein the nonionically and cationically derivatized polygalactomannans are produced by the process comprising the steps of:
(a) contacting guar flour containing nonionic groups with an alcohol or an alcohol/water solution;
(b) neutralizing the reaction mixture of step (a) with an acid;
(c) adding a cationic substituent in an amount such that the resulting material has a degree of substitution of cationic groups ranging from 0.20 to 1.0;
(d) adding an aqueous alkaline solution;
(e) washing the resulting mixture with water, an organic solvent or mixtures thereof; and
(f) recovering the product produced thereby.

2. The polygalactomannans according to claim 1 wherein said nonionic groups comprise hydroxyalkyl groups, wherein alkyl represents a straight or branched hydrocarbon moiety having between 1 and 6 carbon atoms.

3. The polygalactomannans according to claim 2 wherein said nonionic groups are selected from the group consisting of hydroxyethyl and hydroxypropyl.

4. The polygalactomannans according to claim 2 wherein said nonionic groups have a molecular substitution between 0.4 and 1.2.

5. The polygalactomannans according to claim 1 wherein said cationic groups are quaternary groups.

6. The polyglalactomannars according to claim 5 wherein cationic substituents are of the formula 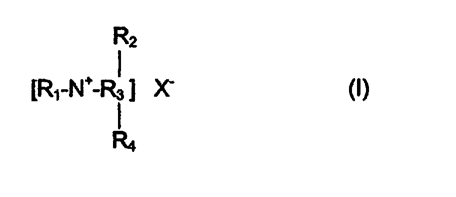 wherein R₁ is a monohydroxylated or polyhydroxylated alkyl group containing between one and six carbon atoms; R₂ and R₃ are independently, alkyl groups containing between one and six carbon atoms; R₄ is an alkyl group containing between one and 24 carbon atoms; and X is a halide.

7. The polygalactomannans according to claim 1 wherein said cationic groups have a degree of substitution between 0.23 and 0.80.

8. The polygalactomannans according to claim 1 wherein said polygalactomannans further comprise anionic groups.

9. The polygalactomannans according to claim 8 wherein said anionic groups comprise carboxyalkyl groups wherein alkyl represents a straight or branched hydrocarbon moiety having between 1 and 6 carbon atoms.

10. The polygalactomannans according to claim 9 wherein said anionic groups comprise carboxymethyl groups.

11. A process for producing polygalactomannans which contain both nonionic and cationic groups and demonstrate greater than 75% light transmission at a wavelength of from 500-600 nanometers when dispersed in water in the amount of 0.5 parts per 100 parts water comprising the steps of:
(a) contacting guar flour containing nonionic groups with an alcohol or an alcohol/water solution;
(b) neutralizing the reaction mixture of step (a) with an acid;
(c) adding a cationic substituent in an amount such that the resulting material has a degree of substitution of cationic groups ranging from 0.20 to 1.0;
(d) adding an aqueous alkaline solution;
(e) washing the resulting mixture with water, an organic solvent or mixtures thereof; and
(f) recovering the product produced thereby.

12. The process according to claim 11 wherein said nonionic groups comprise hydroxyalkyl groups, wherein alkyl represents a straight or branched hydrocarbon moiety having between 1 and 6 carbon atoms.

13. The process according to claim 12 wherein said nonionic groups have a molecular substitution between 0.4 and 1.2.

14. The process according to claim 11 wherein said cationic groups are quaternary groups.

15. The process according to claim 14 wherein cationic substituents are of the formula 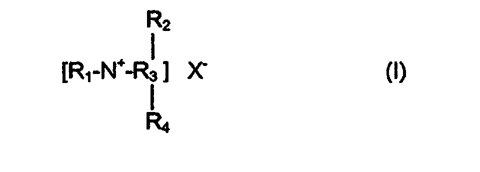 wherein R₁ is a monohydroxylated or polyhydroxylated alkyl group containing between one and six carbon atoms; R₂ and R₃ are independently, alkyl groups containing between one and six carbon atoms; R₄ is an alkyl group containing between one and 24 carbon atoms; and X is a halide.

16. The process according to claim 14 wherein said cationic groups have a degree of substitution between 0.23 and 0.80.

17. The process according to claim 11 wherein said polygalactomannans further comprise anionic groups.

18. The process according to claim 17 wherein said anionic groups comprise carboxyalkyl groups wherein alkyl represents a straight or branched hydrocarbon moiety having between 1 and 6 carbon atoms.

19. An oil field chemical, personal care chemical, industrial cleaning chemical, textile chemical, paper chemical or coating composition including polygalactomannans which contain both nonionic and cationic groups and demonstrate greater than 75% light transmission at a wavelength of from 500-600 nanometers when dispersed in water in the amount of 0.5 parts per 100 parts water wherein the nonionically and cationically derivatized polygalactomannans are produced by the process comprising the steps of:
(a) contacting guar flour containing nonionic groups with an alcohol or an alcohol/water solution;
(b) neutralizing the reaction mixture of step (a) with an acid;
(c) adding a cationic substituent in an amount such that the resulting material has a degree of substitution of cationic groups ranging from 0.20 to 1.0;
(d) adding an aqueous alkaline solution;
(e) washing the resulting mixture with water, an organic solvent or mixtures thereof; and
(f) recovering the product produced thereby.

20. The composition according to claim 19 having a greater than 90% light transmission at a wavelength of from 500-600 nanometers

21. A detergent composition useful for cleaning and/or conditioning human hair or skin or deaning dishes or clothing which contains or NH₄Cl and further contains a nonionically and cationically derivatized polygalactomannan, the molecular substitution of the nonionic substituent being between 0.4 and 1.2 and the degree of substitution of the cationic substituent being between 0.20 to 1.0, said detergent composition having a viscosity of greater than 500 centipoises and said composition having greater than 75% light transmission at a wavelength of from 500-600 nanometers.

22. The composition according to claim 21 which is selected from the group consisting of personal care compositions and industrial cleaning compositions.

23. The composition according to claim 22 comprising a personal care composition useful for cleaning and/or conditioning human hair or skin.

24. The composition according to claim 23 wherein the composition includes NaCl present in an amount of between 0.5 and 3.0 percent by weight of the composition.

25. The composition according to claim 21 wherein said nonionic substituent comprises hydroxyalkyl groups, wherein alkyl represents a straight or branched hydrocarbon moiety having between 1 and 6 carbon atoms.

26. The composition according to claim 25 wherein said nonionic substituents are selected from the group consisting of hydroxyethyl and hydroxypropyl.

27. The composition according to claim 21 wherein said cationic groups are quaternary groups.

28. The composition according to claim 27 wherein said cationic substituents are of the formula 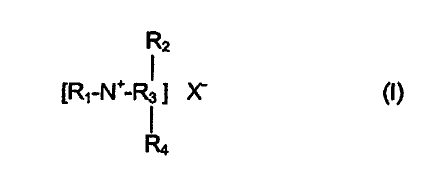 wherein R₁ is a monohydroxylated or polyhydroxylated alkyl group containing between one and six carbon atoms; R₂ and R₃ are independently, alkyl groups containing between one and six carbon atoms; R₄ is an alkyl group containing between one and 24 carbon atoms; and X is a halide.

29. The composition according to claim 21 wherein said cationic substituents have a degree of substitution between 0.23 and 0.80.

30. The composition according to claim 21 wherein said nonionically and cationically derivatized polygalactomannan is present in an amount of 0.1 and 2.0 percent by weight of the composition.

31. The composition according to claim 21 having a greater than 90% light transmission at a wavelength of from 500-600 nanometers.

32. A detergent composition useful for cleaning and/or conditioning human hair or skin or cleaning dishes or clothing which contains NaCl or NH₄Cl and further contains a nonionically, anionically and cationically derivatized polygalactomannan, the molecular substitution of the nonionic substituent being between 0.14 and 1.2, the degree of substitution of the anionic substituent being between 0.10 to 0.30 and the degree of substitution of the cationic substituent being between 0.20 to 1.0, said composition having a viscosity of greater than 500 centipoises and said composition having greater than 75% light transmission at a wavelength of from 500-600 nanometers.

33. The composition according to claim 32 which is selected from the group consisting of personal care compositions and industrial cleaning compositions.

34. The composition according to claim 33 comprising a personal care composition useful for cleaning and/or conditioning human hair or skin.

35. The composition according to claim 34 wherein the composition indudes NaCl present in an amount of between 0.5 and 3.0 percent by weight of the composition.

36. The composition according to claim 32 wherein said nonionic substituent comprises hydroxyalkyl groups, wherein alkyl represents a straight or branched hydrocarbon moiety having between 1 and 6 carbon atoms.

37. The composition according to claim 36 wherein said nonionic substituents are selected from the group consisting of hydroxyethyl and hydroxypropyl.

38. The composition according to claim 32 wherein said cationic groups are quaternary groups.

39. The composition according to claim 38 wherein said cationic substituents are of the formula 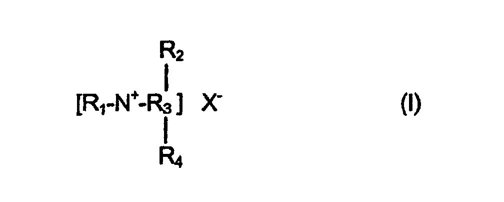 wherein R₁ is a monohydroxylated or polyhydroxylated alkyl group containing between one and six carbon atoms; R₂ and R₃ are independently, alkyl groups containing between one and six carbon atoms; R₄ is an alkyl group containing between one and 24 carbon atoms; and X is a halide.

40. The composition according to claim 32 wherein said cationic substituents have a degree of substitution between 0.23 and 0.80.

41. The composition according to claim 32 wherein said anionic groups comprise carboxyalkyl groups wherein alkyl represents a straight or branched hydrocarbon moiety having between 1 and 6 carbon atoms.

42. The composition according to claim 41 wherein said anionic groups comprise carboxymethyl groups.

43. The composition according to claim 32 having a greater than 90% light transmission at a wavelength of from 500-600 nanometers.

## Patentansprüche

1. Polygalactomannane, welche sowohl nichtionische als auch kationische Gruppen enthalten und bei einer Wellenlänge von 500 - 600 nm eine Uchtdurchlässigkeit von größer 75 % zeigen, wenn sie in Wasser in der Menge von 0,5 Teilen pro 100 Teilen Wasser dispergiert sind, wobei die nichtionisch und kationisch derivatisierten Polygalactomannane durch das Verfahren hergestellt werden, welches die folgenden Schritte umfasst:
(a) Inkontaktbringen von Guarmehl, das nichtionische Gruppen enthält, mit einem Alkohol oder einer Alkohol/Wasserlösung;
(b) Neutralisieren der Reaktionsmischung aus Schritt (a) mit einer Säure;
(c) Zugeben eines kationischen Substituenten in einer solchen Menge, dass das resultierende Material einen Substitutionsgrad an kationischen Gruppen aufweist, der von 0,20 bis 1,0 reicht;
(d) Zugeben einer wässrigen alkalischen Lösung;
(e) Waschen der resultierenden Mischung mit Wasser, einem organischen Lösungsmittel oder Mischungen von diesen; und
(f) Gewinnen des dadurch erzeugten Produkts.

2. Die Polygalactomannane gemäß Anspruch 1, wobei die nichtionischen Gruppen Hydroxyalkylgruppen umfassen, wobei Alkyl für einen geraden oder verzweigten Kohlenwasserstoffanteil mit zwischen 1 und 6 Kohlenstoffatomen steht.

3. Die Polygalactomannane gemäß Anspruch 2, wobei die nichtionischen Gruppen ausgewählt sind aus der Gruppe bestehend aus Hydroxyethyl und Hydroxypropyl.

4. Die Polygalactomannane gemäß Anspruch 2, wobei die nichtionischen Gruppen eine molekulare Substitution zwischen 0,4 und 1,2 aufweisen.

5. Die Polygalactomannane gemäß Anspruch 1, wobei die kationischen Gruppen quartäre Gruppen sind.

6. Die Polygalactomannane gemäß Anspruch 5, wobei kationische Substituenten die Formel 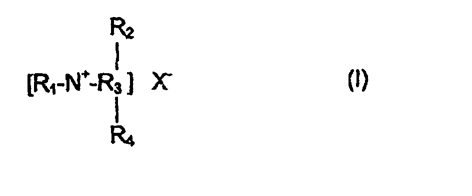 aufweisen, worin R₁ eine monohydroxylierte oder polyhydroxylierte Alkylgruppe ist, die zwischen 1 und 6 Kohlenstoffatome enthält; R₂ und R₃ unabhängig Alkylgruppen sind, die zwischen 1 und 6 Kohlenstoffatome enthalten; R₄ eine Alkylgruppe ist, die zwischen 1 und 24 Kohlenstoffatome enthält; und X ein Halogenid ist.

7. Die Polygalactomannane gemäß Anspruch 1, wobei die kationischen Gruppen einen Substitutionsgrad zwischen 0,23 und 0,80 aufweisen.

8. Die Polygalactomannane gemäß Anspruch 1, wobei die Polygalactomannane weiterhin anionische Gruppen umfassen.

9. Die Polygalactomannane gemäß Anspruch 8, wobei die anionischen Gruppen Carboxyalkylgruppen umfassen, wobei Alkyl für einen geraden oder verzweigten Kohlenwasserstoffanteil mit zwischen 1 und 6 Kohlenstoffatomen steht.

10. Die Polygalactomannane gemäß Anspruch 9, wobei die anionischen Gruppen Carboxymethylgruppen umfassen.

11. Ein Verfahren zur Herstellung von Polygalactomannanen, welche sowohl nichtionische als auch kationische Gruppen enthalten und eine Lichtdurchlässigkeit bei einer Wellenlänge von 500 - 600 nm von größer 75 % zeigen, wenn sie in Wasser in der Menge von 0,5 Teilen pro 100 Teilen Wasser dispergiert sind, welches die folgenden Schritte umfasst:
(a) Inkontaktbringen von Guarmehl, das nichtionische Gruppen enthält, mit einem Alkohol oder einer Alkohol/Wasserlösung;
(b) Neutralisieren der Reaktionsmischung aus Schritt (a) mit einer Säure;
(c) Zugeben eines kationischen Substituenten in einer solchen Menge, dass das resultierende Material einen Substitutionsgrad an kationischen Gruppen aufweist, der von 0,20 bis 1,0 reicht;
(d) Zugeben einer wässrigen alkalischen Lösung;
(e) Waschen der resultierenden Mischung mit Wasser, einem organischen Lösungsmittel oder Mischungen von diesen; und
(f) Gewinnen des dadurch erzeugten Produkts.

12. Das Verfahren gemäß Anspruch 11, wobei die nichtionischen Gruppen Hydroxyalkylgruppen umfassen, wobei Alkyl für einen geraden oder verzweigten Kohlenwasserstoffänteil mit zwischen 1 und 6 Kohlenstoffatomen steht.

13. Das Verfahren gemäß Anspruch 12, wobei die nichtionischen Gruppen eine molekulare Substitution zwischen 0,4 und 1,2 aufweisen.

14. Das Verfahren gemäß Anspruch 11, wobei die kationischen Gruppen quartäre Gruppen sind.

15. Das Verfahren gemäß Anspruch 14, wobei kationische Substituenten die Formel 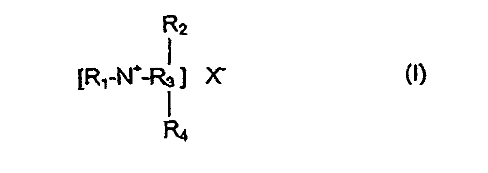 aufweisen, worin R₁ eine monohydroxylierte oder polyhydroxylierte Alkylgruppe ist, die zwischen 1 und 6 Kohlenstoffatome enthält; R₂ und R₃ unabhängig Alkylgruppen sind, die zwischen 1 und 6 Kohlenstoffatome enthalten; R₄ eine Alkylgruppe ist, die zwischen 1 und 24 Kohlenstoffatome enthält; und X ein Halogenid ist.

16. Das Verfahren gemäß Anspruch 14, wobei die kationischen Gruppen einen Substitutionsgrad zwischen 0,23 und 0,80 aufweisen.

17. Das Verfahren gemäß Anspruch 11, wobei die Polygalactomannane darüber hinaus anionische Gruppen umfassen.

18. Das Verfahren gemäß Anspruch 17, wobei die anionischen Gruppen Carboxyalkylgruppen umfassen, wobei Alkyl für einen geraden oder verzweigten Kohlenwasserstoffanteil mit zwischen 1 und 6 Kohlenstoffatomen steht.

19. Eine Ölfeldchemikalie, eine Chemikalie zur Körperpflege, eine industrielle Reinigungschemikalie, eine Textilchemikalie, Papierchemikalie oder Beschichtungszusammensetzung, welche Polygalactomannane einschließt, welche sowohl nichtionische als auch kationische Gruppen enthalten und eine Lichtdurchlässigkeit bei einer Wellenlänge von 500 - 600 nm von größer 75 % zeigen, wenn sie in Wasser in der Menge von 0,5 Teilen pro 100 Teilen Wasser dispergiert sind, wobei die nichtionisch und kationisch derivatisierten Polygalactomannane durch das Verfahren hergestellt werden, welches die folgenden Schritte umfasst:
(a) Inkontaktbringen von Guarmehl, das nichtionische Gruppen enthält, mit einem Alkohol oder einer Alkohol/Wasserlösung;
(b) Neutralisieren der Reaktionsmischung aus Schritt (a) mit einer Säure;
(c) Zugeben eines kationischen Substituenten in einer solchen Menge, dass das resultierende Material einen Substitutionsgrad an kationischen Gruppen aufweist, der von 0,20 bis 1,0 reicht;
(d) Zugeben einer wässrigen alkalischen Lösung;
(e) Waschen der resultierenden Mischung mit Wasser, einem organischen Lösungsmittel oder Mischungen von diesen; und
(f) Gewinnen des dadurch erzeugten Produkts.

20. Die Zusammensetzung gemäß Anspruch 19 mit einer Lichtdurchlässigkeit bei einer Wellenlänge von 500 - 600 nm von größer 90 %.

21. Eine Detergenzzusammensetzung, die zum Reinigen und/oder Konditionieren von menschlichem Haar oder Haut oder zum Reinigen von Geschirr oder Kleidung nützlich ist, welche NaCl oder NH₄Cl enthält und darüber hinaus ein nichtionisch und kationisch derivatisiertes Polygalactomannan enthält, wobei die molekulare Substitution des nichtionischen Substituenten zwischen 0,4 und 1,2 liegt und der Substitutionsgrad des kationischen Substituenten zwischen 0,20 bis 1,0 liegt, wobei die Detergenzzusammensetzung eine Viskosität von größer 500 Centipoise aufweist und die Zusammensetzung eine Lichtdurchlässigkeit bei einer Wellenlänge von 500 - 600 nm von größer 75 % aufweist.

22. Die Zusammensetzung gemäß Anspruch 21, welche ausgewählt ist aus der Gruppe bestehend aus Zusammensetzungen zur Körperpflege und industriellen Reinigungszusammensetzungen.

23. Die Zusammensetzung gemäß Anspruch 22, welche eine Zusammensetzung zur Körperpflege umfasst, die zum Reinigen und/oder Konditionieren von menschlichem Haar oder Haut nützlich ist.

24. Die Zusammensetzung gemäß Anspruch 23, wobei die Zusammensetzung NaCl einschließt, das in einer Menge zwischen 0,5 und 3,0 Gew.-% der Zusammensetzung vorliegt.

25. Die Zusammensetzung gemäß Anspruch 21, wobei der nichtionische Substituent Hydroxyalkylgruppen umfasst, wobei Alkyl für einen geraden oder verzweigten Kohlenwasserstoffanteil mit zwischen 1 und 6 Kohlenstoffatomen steht.

26. Die Zusammensetzung gemäß Anspruch 25, wobei die nichtionischen Substituenten ausgewählt sind aus der Gruppe bestehend aus Hydroxyethyl und Hydroxypropyl.

27. Die Zusammensetzung gemäß Anspruch 21, wobei die kationischen Gruppen quartäre Gruppen sind.

28. Die Zusammensetzung gemäß Anspruch 27, wobei die kationischen Substituenten die Formel 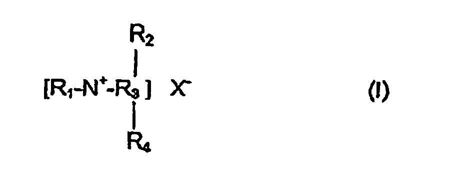 aufweisen, worin R₁ eine monohydroxylierte oder polyhydroxylierte Alkylgruppe ist, die zwischen 1 und 6 Kohlenstoffatome enthält; R₂ und R₃ unabhängig Alkylgruppen sind, die zwischen 1 und 6 Kohlenstoffatome enthalten; R₄ eine Alkylgruppe ist, die zwischen 1 und 24 Kohlenstoffatome enthält; und X ein Halogenid ist.

29. Die Zusammensetzung gemäß Anspruch 21, wobei die kationischen Substituenten einen Substitutionsgrad zwischen 0,23 und 0,80 aufweisen.

30. Die Zusammensetzung gemäß Anspruch 21, wobei das nichtionisch und kationisch derivatisierte Polygalactomannan in einer Menge von 0,1 und 2,0 Gew.-% der Zusammensetzung vorliegt.

31. Die Zusammensetzung gemäß Anspruch 21, welche eine Lichtdurchlässigkeit bei einer Wellenlänge von 500 - 600 nm von größer 90 % aufweist.

32. Eine Detergenzzusammensetzung, die zum Reinigen und/oder Konditionieren von menschlichem Haar oder Haut oder zum Reinigen von Geschirr oder Kleidung nützlich ist, welche NaCl oder NH₄Cl enthält und darüber hinaus ein nichtionisch, anionisch und kationisch derivatisiertes Polygalactomannan enthält, wobei die molekulare Substitution des nichtionischen Substituenten zwischen 0,14 und 1,2 liegt, der Substitutionsgrad des anionischen Substituenten zwischen 0,10 bis 0,30 liegt und der Substitutionsgrad des kationischen Substituenten zwischen 0,20 bis 1,0 liegt, wobei die Zusammensetzung eine Viskosität von größer 500 Centipoise aufweist und die Zusammensetzung eine Lichtdurchlässigkeit bei einer Wellenlänge von 500 - 600 nm von größer 75 % aufweist.

33. Die Zusammensetzung gemäß Anspruch 32, welche ausgewählt ist aus der Gruppe bestehend aus Zusammensetzungen zur Körperpflege und industriellen Reinigungszusammensetzungen.

34. Die Zusammensetzung gemäß Anspruch 33, welche eine Zusammensetzung zur Körperpflege umfasst, die zum Reinigen und/oder Konditionieren von menschlichem Haar oder Haut nützlich ist.

35. Die Zusammensetzung gemäß Anspruch 34, wobei die Zusammensetzung NaCl einschließt, das in einer Menge zwischen 0,5 und 3,0 Gew.-% der Zusammensetzung vorliegt.

36. Die Zusammensetzung gemäß Anspruch 32, wobei der nichtionische Substituent Hydroxyalkylgruppen umfasst, wobei Alkyl für einen geraden oder verzweigten Kohlenwasserstoffanteil mit zwischen 1 und 6 Kohlenstoffatomen steht.

37. Die Zusammensetzung gemäß Anspruch 36, wobei die nichtionischen Substituenten ausgewählt sind aus der Gruppe bestehend aus Hydroxyethyl und Hydroxypropyl.

38. Die Zusammensetzung gemäß Anspruch 32, wobei die kationischen Gruppen quartäre Gruppen sind.

39. Die Zusammensetzung gemäß Anspruch 38, wobei die kationischen Substituenten die Formel 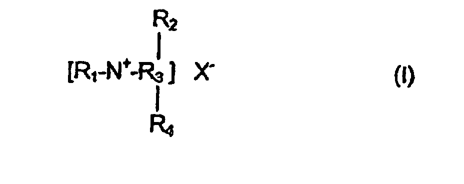 aufweisen, worin R₁ eine monohydroxylierte oder polyhydroxylierte Alkylgruppe ist, die zwischen 1 und 6 Kohlenstoffatome enthält; R₂ und R₃ unabhängig Alkylgruppen sind, die zwischen 1 und 6 Kohlenstoffatome enthalten; R₄ eine Alkylgruppe ist, die zwischen 1 und 24 Kohlenstoffatome enthält; und X ein Halogenid ist.

40. Die Zusammensetzung gemäß Anspruch 32, wobei die kationischen Substituenten einen Substitutionsgrad zwischen 0,23 und 0,80 aufweisen.

41. Die Zusammensetzung gemäß Anspruch 32, wobei die anionischen Gruppen Carboxyalkylgruppen umfassen, wobei Alkyl für einen geraden oder verzweigten Kohlenwasserstoffanteil mit zwischen 1 und 6 Kohlenstoffatomen steht.

42. Die Zusammensetzung gemäß Anspruch 41, wobei die anionischen Gruppen Carboxymethylgruppen umfassen.

43. Die Zusammensetzung gemäß Anspruch 32, welche eine Lichtdurchlässigkeit bei einer Wellenlänge von 500 - 600 nm von größer 90 % aufweist.

## Revendications

1. Polygalactomannanes contenant à la fois des groupes non ioniques et cationiques et possédant un coefficient de transmission de la lumière supérieur à 75 % à une longueur d'onde de 500 à 600 nanomètres quand ils sont dispersés dans de l'eau à une teneur de 0,5 partie pour 100 parties d'eau, dans lesquels les polygalactomannanes fonctionnalisés par des groupes non ioniques et cationiques sont préparés selon un procédé comprenant les étapes consistant à :
(a) mettre en contact de la farine de guar contenant des groupes non ioniques avec un alcool ou une solution alcool/eau;
(b) neutraliser le mélange réactionnel de l'étape (a) avec un acide ;
(c) ajouter un substituant cationique en une quantité telle que le matériau résultant possède un taux de substitution de groupes cationiques compris entre 0,20 et 1,0;
(d) ajouter une solution aqueuse alcaline ;
(e) laver le mélange résultant avec de l'eau, un solvant organique, ou des mélanges de ceux-ci ; et
(f) récupérer le produit ainsi obtenu.

2. Polygalactomannanes selon la revendication 1, dans lesquels lesdits groupes non ioniques comprennent des groupes hydroxyalkyle, où l'alkyle désigne un radical hydrocarboné linéaire ou ramifié comportant de 1 à 6 atomes de carbone.

3. Polygalactomannanes selon la revendication 2, dans lesquels lesdits groupes non ioniques sont choisis parmi les groupes hydroxyéthyle et hydroxypropyle.

4. Polygalactomannanes selon la revendication 2, dans lesquels lesdits groupes non ioniques ont un taux de substitution moléculaire compris entre 0,4 et 1,2.

5. Polygalactomannanes selon la revendication 1, dans lesquels lesdits groupes cationiques sont des groupes quaternaires.

6. Polygalactomannanes selon la revendication 5, dans lesquels les substituants cationiques ont la formule suivante : 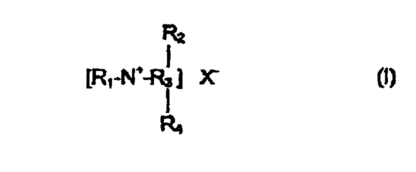 dans laquelle R₁ est un groupe alkyle monohydroxylé ou polyhydroxylé contenant de 1 à 6 atomes de carbone ; R2 et R3 sont, de façon indépendante, des groupes alkyle contenant de 1 à 6 atomes de carbone ; R4 est un groupe alkyle contenant de 1 à 24 atomes de carbone ; et X est un halogénure.

7. Polygalactomannanes selon la revendication 1, dans lesquels lesdits groupes cationiques ont un taux de substitution compris entre 0,23 et 0,80.

8. Polygalactomannanes selon la revendication 1, dans lesquels lesdits polygalactomannanes comprennent en outre des groupes anioniques.

9. Polygalactomannanes selon la revendication 1, dans lesquels lesdits groupes anioniques sont des groupes carboxyalkyle où l'alkyle représente un radical hydrocarboné linéaire ou ramifié comportant de 1 à 6 atomes de carbone.

10. Polygalactomannanes selon la revendication 9, dans lesquels lesdits groupes anioniques sont des groupes carboxyméthyle.

11. Procédé de préparation de polygalactomannanes contenant des groupes non ioniques et cationiques et possédant un coefficient de transmission de la lumière supérieur à 75 % à une longueur d'onde de 500 à 600 manomètres quand ils sont dispersés dans de l'eau à une teneur de 0,5 partie pour 100 parties d'eau, comprenant les étapes consistant à :
(a) mettre en contact de la farine de guar contenant des groupes non ioniques avec un alcool ou une solution alcool/eau ;
(b) neutraliser le mélange réactionnel de l'étape (a) avec un acide ;
(c) ajouter un substituant cationique en une quantité telle que le matériau résultant possède un taux de substitution de groupes cationiques compris entre 0,20 à 1,0 ;
(d) ajouter une solution aqueuse alcaline ;
(e) laver le mélange résultant avec de l'eau, un solvant organique, ou des mélanges de ceux-ci ; et
(f) récupérer le produit ainsi obtenu.

12. Procédé selon la revendication 11, dans lequel lesdits groupes non ioniques sont des groupes hydroxyalkyle, où l'alkyle désigne un radical hydrocarboné linéaire ou ramifié comportant de 1 à 6 atomes de carbone.

13. Procédé selon la revendication 12, dans lequel lesdits groupes non ioniques ont un taux de substitution moléculaire compris entre 0,4 et 1,2.

14. Procédé selon la revendication 11, dans lequel lesdits groupes cationiques sont des groupes quaternaires.

15. Procédé selon la revendication 14, dans lequel les substituants cationiques ont la formule suivante : 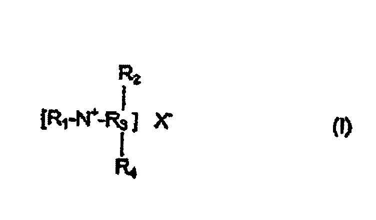 dans laquelle R₁ est un groupe alkyle monohydroxylé ou polyhydroxylé contenant de 1 à 6 atomes de carbone ; R₂ et R₃ sont, de façon indépendante, des groupes alkyle contenant de 1 à 6 atomes de carbone ; R₄ est un groupe alkyle contenant de 1 à 24 atomes de carbone ; et X est un halogénure.

16. Procédé selon la revendication 14, dans lequel lesdits groupes cationiques ont un taux de substitution compris entre 0,23 et 0,80.

17. Procédé selon la revendication 11, dans lequel lesdits polygalactomannanes comprennent en outre des groupes anioniques.

18. Procédé selon la revendication 17, où lesdits groupes anioniques sont des groupes carboxyalkyle, où l'alkyle désigne un radical hydrocarboné linéaire ou ramifié comportant de 1 à 6 atomes de carbone.

19. Produit chimique pour le domaine du pétrole, produit chimique de soin personnel, produit chimique de nettoyage industriel, produit chimique pour le textile, produit chimique pour le papier, ou composition de revêtement comprenant des polygalactomannanes contenant des groupes non ioniques et cationiques possédant un coefficient de transmission de la lumière supérieur à 75 % à 500 à 600 nanomètres quand ils sont dispersés dans de l'eau en une quantité de 0,5 partie par 100 parties d'eau, dans lequel les polygalactomannanes, fonctionnalisés par des groupes non ioniques et cationiques sont préparés selon un procédé comprenant les étapes consistant à :
(a) mettre en contact de la farine de guar contenant des groupes non ioniques avec un alcool ou une solution alcool/eau ;
(b) neutraliser le mélange de réaction de l'étape (a) avec un acide ;
(c) ajouter un substituant cationique en une quantité telle que le matériau résultant possède un taux de substitution de groupes cationiques compris entre 0,20 et 1,0 ;
(d) ajouter une solution alcaline aqueuse ;
(e) laver le mélange résultant avec de l'eau, un solvant organique, ou des mélanges de ceux-ci ; et
(f) récupérer le produit réalisé ainsi obtenu.

20. Composition selon la revendication 19 possédant un coefficient de transmission de la lumière supérieur à 90 % à une longueur d'onde située entre 500 et 600 nanomètres.

21. Composition détergente utile pour le nettoyage et/ou le conditionnement des cheveux ou de la peau chez l'être humain, ou pour le nettoyage de la vaisselle ou de vêtements, qui contient du NaCl ou du HN₄Cl, et qui contient en outre un polygalactomannane fonctionnalisé par des groupes non ioniques et cationiques, le taux de substitution moléculaire du substituant non ionique étant compris entre 0,4 et 1,2 et le taux de substitution du substituant cationique étant compris entre 0,20 et 1,0, ladite composition détergente ayant une viscosité supérieure à 500 centipoises et ladite composition ayant un coefficient de transmission de la lumière supérieur à 75 % à une longueur d'onde située entre 500 et 600 nanomètres.

22. Composition selon la revendication 21, choisie parmi les compositions de soin personnel et les compositions de nettoyage industriel.

23. Composition selon la revendication 22, constituant une composition de soin personnel utile pour le nettoyage et/ou le conditionnement des cheveux ou de la peau chez l'être humain.

24. Composition selon la revendication 23, dans laquelle la composition comprend du NaCl en une quantité pondérale située entre 0,5 et 3,0 pour cent.

25. Composition selon la revendication 21, dans laquelle ledit substituant non ionique comprend des groupes hydroxyalkyle, où l'alkyle désigne un radical hydrocarboné linéaire ou ramifié comportant de 1 à 6 atomes de carbone.

26. Composition selon la revendication 25, dans laquelle lesdits substituants non ioniques sont choisis parmi les groupements hydroxyéthyle et hydroxypropyle.

27. Composition selon la revendication 21, dans laquelle lesdits groupes cationiques sont des groupes quaternaires.

28. Composition selon la revendication 27, dans laquelle lesdits substituants cationiques ont la formule suivante : 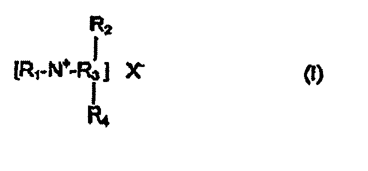 dans laquelle R₁ est un groupe alkyle monohydroxylé ou polyhydroxylé contenant de 1 à 6 atomes de carbone ; R₂ et R₃ sont, indépendamment, des groupes alkyle contenant de 1 à 6 atomes de carbone ; R₄ est un groupe alkyle contenant de 1 à 24 atomes de carbone ; et X est halogénure.

29. Composition selon la revendication 21, dans laquelle lesdits substituants cationiques ont un taux de substitution compris entre 0,23 et 0,80.

30. Composition selon la revendication 21, dans laquelle ledit polygalactomannane fonctionnalisé par des groupes non ioniques et cationiques est présent en une quantité pondérale de 0,1 et 2,0 pour cent de la composition.

31. Composition selon la revendication 21, possédant un coefficient de transmission de la lumière supérieur à 90 % à une longueur d'onde située entre 500 et 800 nanomètres.

32. Composition détergente utile pour le nettoyage et/ou le conditionnement des cheveux ou de la peau chez l'être humain, ou pour le nettoyage de la vaisselle ou de vêtements, qui contient du NaCl ou du NH₄Cl et qui contient en outre un polygalactomannane fonctionnalisé par des groupes non ioniques, anioniques et cationiques, le taux de substitution moléculaire du substituant non ionique se situant entre 0,14 et 1,2, le taux de substitution du substituant anionique se situant entre 0,10 et 0,30, et le taux de substitution du substituant cationique se situant entre 0,20 et 1,0, ladite composition possédant une viscosité supérieure à 500 centipoises et la dite composition possédant un coefficient de transmission de la lumière supérieur à 75 a une longueur d'onde de 500 à 800 nanomètres.

33. Composition selon la revendication 32, choisie parmi les compositions de soin personnel et les compositions de nettoyage industriel.

34. Composition selon la revendication 33, constituant une composition de soin personnel utile pour le nettoyage et/ou le conditionnement de cheveux ou de la peau chez l'être humain.

35. Composition selon la revendication 34, dans laquelle la composition comprend du NaCl présent en une quantité pondérale située entre 0,5 et 3,0 pour cent de la composition.

36. Composition selon la revendication 32, dans laquelle ledit substituant non ionique comprend des groupes hydroxyalkyle, dans laquelle l'alkyle désigne un radical hydrocarboné linéaire ou ramifié comportant de 1 à 6 atomes de carbone.

37. Composition selon la revendication 38, dans laquelle lesdits substituants non ioniques sont choisis parmi hydroxyéthyle et hydroxypropyle.

38. Composition selon la revendication 32, dans laquelle lesdits groupes cationiques sont des groupes quaternaires.

39. Composition selon la revendication 38, dans laquelle lesdits substituants cationiques ont la formule suivante : 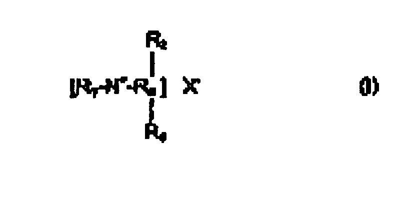 dans laquelle R₁ est un groupe alkyle monohydroxylé ou polyhydroxylé contenant de 1 à 6 atomes de carbone ; R₂ et R₃ sont, indépendamment, des groupes alkyle contenant de 1 à 6 atomes de carbone ; R₄ est un groupe alkyle contenant de 1 à 24 atomes de carbone ; et X est un halogénure.

40. Composition selon la revendication 32, dans laquelle lesdits substituants cationiques ont un taux de substitution situé entre 0,23 et 0,80.

41. Composition selon la revendication 32, dans laquelle lesdits groupes cationiques sont des groupes caboxyalkyle, où l'alkyle désigne un radical hydrocarboné linéaire ou ramifié comportant de 1 à 6 atomes de carbone.

42. Composition selon la revendication 41, dans laquelle lesdits groupes anioniques sont des groupes carboxyméthyle.

43. Composition selon la revendication 32, possédant un coefficient de transmission de la lumière supérieur à 90 % pour une longueur d'onde située entre 500 et 800 nanomètres.
